# NEW EUROPEAN PATENT SPECIFICATION

(11) **EP 1 060 241 B2**
(45) Date of publication and mention of the opposition decision: **20.10.2010**
(45) Mention of the grant of the patent: 07.03.2007
(21) Application number: 98966693.8
(22) Date of filing: 17.12.1998
(51) Int. Cl.: C12N 5/00, C12N 7/00

(54) **PREPARATION OF CELLS FOR PRODUCTION OF BIOLOGICALS**
HERSTELLUNG VON ZELLEN FÜR DIE PRODUKTION VON BIOLOGISCHEN PRODUKTEN
PREPARATION DE CELLULES POUR LA PRODUCTION DE SUBSTANCES BIOLOGIQUES

(30) Priority: 24.12.1997 EP 97204110
(43) Date of publication of application: 20.12.2000
(60) Divisional application: 07103139.7
(73) Proprietor: Solvay Biologicals B.V., 1381 CP Weesp (NL)
(72) Inventor: BRANDS, Rudi, Duphar International Research B.V., NL-1381 CP Weesp (NL)
(74) Representative: Prüfer & Partner GbR European Patent Attorneys
(86) International application number: PCT/EP1998/008522
(87) International publication number: WO 1999/033955

(56) References cited:
- EP-A- 0 417 531
- WO-A-89/08701
- WO-A-92/10564

## Description

The present invention is concerned with a method for the preparation of cells for use in the production of biologicals.

For the production of biologicals in e.g. cell lines, the preparation of large amounts of cells using an scaling up procedure in bioreactors will be necessary.

The US patent No. 5,017,490 discloses such a scaling up procedure which provides in particular the advantage of a low risk of transfer contamination. This method is, however, not suited for anchorage dependent cells (hence, not for cells which only grow if fixed to a substrate) or cells embedded in a substrate (e.g. in porous carriers).

The US patent No. 4,644,912 discloses a method for the preparation of anchorage-dependent cells for the production of biologicals (i.e. viruses) starting with a cell working seed, and with subsequent passages effected in increasing consecutive volumes of 1 litre, 5 litre, 25 litre, 150 litre bioreactors, and finally either in a 1000 litre bioreactor or in a multiplicity of 150 litre bioreactors. In between any of these passage steps the cells were released from their carriers with a dilute protease solution. In the final passage the inoculation by the virus was effected.

Assuming average cell cycle times of about 20-24 hours the passage intervals may be about every 3-5 day. Therefore, in order to expand the cells to sufficient large cultures from a MWCS¹ the total scaling up procedure may take several weeks, depending on the final bioreactor volume.
¹MWCS = manufacturer's working cell bank

In the above methods for preparation of cells each of the ultimate production batches has to be prepared from the MWCS. For the production of vast amounts of biologicals it will be necessary to utilise several parallel culturing lines up to the largest vessel volumes. Such preparation procedure, hence, is very time consuming and necessitates the operation of a very considerable number of bioreactors for the preparation of the cells as well as for the production of the biologicals.

It is an object of the present invention to provide a much faster through-put in preparation of cells for the production of biologicals.

Accordingly, the present invention relates to a method for the preparation of cells for use in the production of biologicals, by culturing cells up till a desired cell volume of a preproduction batch, where after in a repeated discontinuous process:
a) part of the cells of the preproduction batch is used for the preparation of at least one production batch, and
b) the remaining part of the cells of the preproduction batch is used as a seed for the preparation of at least one subsequent preproduction batch.
wherein the cells are anchorage-dependent cells which for their growing and/or propagation need to be attached to a substrate.

More in particular, the present invention relates to a method for the preparation of such anchorage-dependent cells for use in the production of biologicals, by culturing cells up till a desired cell volume of a preproduction batch, where after in a repeated discontinuous process:
a) part of the cells of the preproduction batch is transferred to be used for the preparation of at least one production batch, and
b) the remaining part of the cells of the preproduction batch is transferred to be used as a seed for the preparation of at least one subsequent preproduction batch.

In a preferred embodiment of the present invention the first preproduction batch is prepared from a working seed stock by at least one passage step.

According to the present invention the cells which are prepared are anchorage-dependent. It will then be advisable during the repeated process each time when part of a batch is used for the preparation of a new batch to add an additional amount of substrate. In a preferred embodiment, each time prior to the addition of substrate at least part of the cells are first released from their original substrate

As used herein the expression "production batch" means a culture of cells which is employed for the production of biologicals.

As used herein the expression "preproduction batch" means a culture of cells which is used in the process according to the present invention for the preparation of at least one production batch (as defined above) and one subsequent preproduction batch.

As used herein the expression "biological" means any substance or organism which can be produced from a cell culture. Examples of "biologicals" are viruses and proteins such as enzymes.

As used herein the expression "working seed stock" means an amount of a certain type of cells of defined ancestry stored to be used as a seed from which all cultures of the same type of cells are derived.

As used herein the expression "anchorage-dependent cells" means cells which for their proper growing and/or propagation need to be attached to a substrate as defined herein.

As used herein the expression "substrate" means any particulate matter useful for the attachment of cells.

As used herein the expression "passage step" means a sequence of activities in the propagation and production of cells comprising at least the transfer of a suitable amount of cells and of a suitable amount of culturing medium into a production vessel, the incubation of the vessel at conditions suitable for the growing and propagation of the cells during a time sufficient for effective growing and propagation of the cells. Optionally a passage step may comprise separation of the cells from the culture medium and/or from the substrate after a time sufficient for effective growing and propagation of the cells.

It will be clear to the man skilled in the art that the method according to the present invention differs essentially from methods known in the art wherein cells are produced in a continuous process rather than the present discontinuous process. According to the patent publications EP0417531 and WO89/08701 continuous culture systems can be employed for the production of viruses as well. Firstly cells are grown in a first bioreactor, and after a certain cell density is reached cells are fed continuously from said first bioreactor into a second bioreactor. In this second bioreactor viruses are grown on the cells and subsequently these viruses are withdrawn continuously from this second bioreactor.

The basic method of working according to the present invention is to use a mother bioreactor from which the production bioreactor(s) is (are) fed with cells. The cells are anchorage dependent, so after each passage step cells preferably need to be detached from their substrates.
A trypsinisation procedure on large bioreactors has been developed for this purpose.
The production cells are defined up to a specific and characterised passage number for a so-called ECB². The method described allows high through-put production since the up scaling route from WCS to production cells can be very much shortened and much less bioreactors are needed since parallel production lines are not needed anymore.
² ECB = Extended Cell Bank

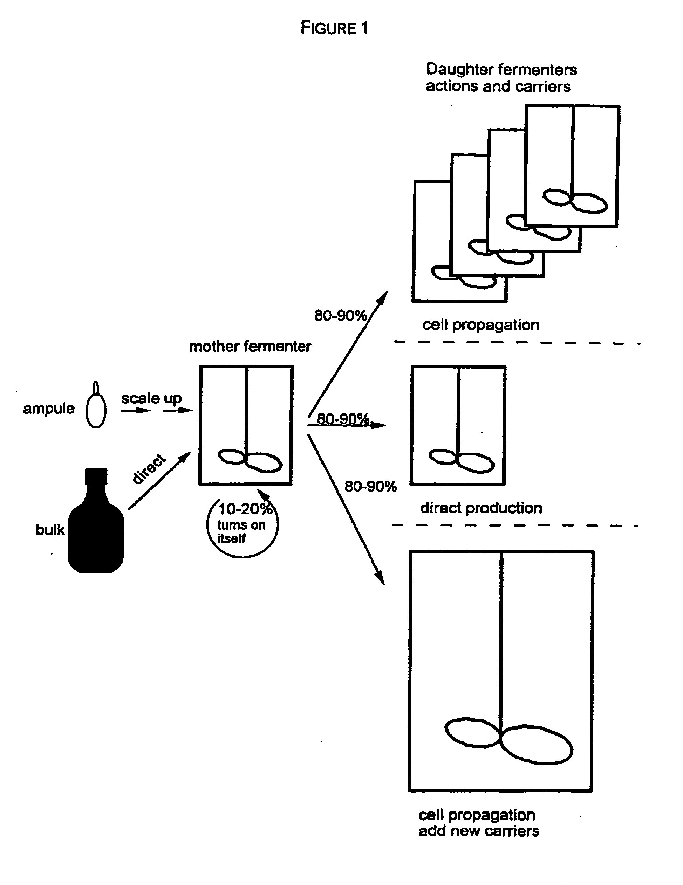

Various embodiments of the present invention are depicted in Figure 1.

In a preferred embodiment cells are expanded from one ampoule of a MWCS up to the level of the first preproduction batch through one or more passage steps. The size of the bioreactor used for such a preproduction batch can range from several litres working volume to several hundreds of litres. Next, a part e.g. 10-20 % of the cells thus expanded (e.g. passage X) are used to repopulate a bioreactor for the production of a subsequent preproduction batch (being passage number X+1), whereas the bulk of the cells is transferred (passage X or X+1) to a larger bioreactor size in order to start production directly or to first populate it, and subsequently start production.

In classical serial production lines the number of doubling of the cells derived from the MWCS at the moment of harvest is known up front within certain limits. A maximum allowable generation number is set to the production system at the onset.

In the method according to the present invention the maximum number of cell passages can be defined by ECB. Production passage number (the number of cell passages used prior to production of the biological product), hence, is irrelevant within the limits set by ECB. As a consequence, such maximum number of passages is to be obeyed in view of regulatory restrictions. As a result the particular batch of produced biologicals is the end product of one direct scaling up route.

In order to verify whether the specifications of the cells at the stage of ECB in production are similar to the MCB³ one need to perform specific validation for this purpose with respect to growth characteristics, freedom of adventitious, extraneous and endogenous agents at the different stages, karyology, iso-enzyme analysis and so on. Once such ECB is fully characterised one may allow to produce the product with cells at any passage number between MCB and ECB, since it may be assumed that cells have not changed in between in their specs. As a result tests on the MWCS therefore can be limited to sterility testing. This is a particular advantage of the method according to the present invention.
³ MCB = Master Cell Bank

With the maximum passage number set one may use cells at any stage in between. From this in order to further minimise the time needed to expand the cells from the MWCS to production bioreactor it would be an advantage to enable bulk start-up of cells. This can be done for example in one of the following ways:
- Cells may be parked at a certain passage number during longer intervals at ambient temperature (17-32 °C) and be revitalised to log expansion growth by raising the temperature and changing the culture medium, or
- Cells may be frozen (Temp < -80°C) in bulk and be thawed prior to transfer them to a preset volume bioreactor, thereby reducing the needed up scaling route significantly.

The method according to the present invention can be carried out with anchorage dependent cells from animal cell cultures. Suitable types of cells are e.g. hamster cells (CHO, BHK-1), monkey cells (Vero), bovine cells (MDBK), canine cells (MDCK), human cells (CaCo, A431) or chicken cells (CEF).

As a bioreactor according to the present inventions can be used a single unit of a plurality of units of e.g. stirred fermenters, fixed bed fermenters, fluidized bed fermenters, air lift fermenters, or a hollow fibre reactors.

Cells of the above types can and some even should be cultured when fixed to a solid support, like micro-carriers or macro-carriers in suspension, e.g. in a fixed bed, a fluidized bed or in suspension, or like hollow fibres. Cells can also be embedded into a carrier (e.g. porous carrier)

In the course of the method according to the present invention, using a solid support, cells are to be released from this solid support. This can be effected by any method useful for detaching of cells from a solid support. Advantageously, to this end use can be made of a proteolytic enzyme solution. Optionally, this enzymatic release step can be preceded by one or more pre-conditioning steps, e.g. by treatment with PBS and/or EDTA, in order to enhance the proteolytic efficiency, and/or in order to reduce the amount of proteolytic enzyme required.

### EXAMPLE 1

### Cell detachment and separation from carriers prior to transfer to next bioreactor

Anchorage dependent cells of a MDCK⁴ cell line were cultured at 37 °C on Cytodex-3 micro carriers (Pharmacia, Uppsala, Sweden) (5 g of carriers/1) in a stirred bioreactor of 4 litre ("mother bioreactor"). The growth medium was EpiSerf (Life Technologies, Paisly, Scotland). Growth was continued till a maximum of 5x10⁶ cells/ml of culture.
The cells were detached from the carriers by trypsinisation in a Trypsin-EDTA solution ( Life Technologies, Paisly, Scotland).
After settling of the carriers 80% of the detached cells were transferred to 3 other bioreactors of similar size. The latter "production" bioreactors all have carriers (cell substrate) added to them up front. Cells were allowed to repopulate the carriers and subsequently used for production in these production bioreactors.
The remainder of the cells in the "mother bioreactor" were allowed to repopulate the remaining Cytodex-3 carriers and were cultured to the desired cell density.
⁴ MDCK = Madin Darby Canine Kidney (cell line)

### EXAMPLE 2

### Cell detachment without separation from carriers prior to transfer to next bioreactor

The culturing of cells was carried out as described in Example 1, however after trypsinisation 80% of the detached cells including the carriers are transferred to the 3 production bioreactors. Additionally, suitable carriers were added to all bioreactors.

### EXAMPLE 3

### Cell detachment without separation from carriers after transfer to next bioreactor

The culturing of cells was carried out as described in Example 1, however, 80 % of still adhered cells were transferred to a bioreactor of similar size which next was used directly for product generation.
The remaining cells on micro carriers in the mother fermenter were next detached by trypsinisation, where after new carriers were added and cells were allowed to repopulate the substrates.

### EXAMPLE 4

### Start-up from frozen bulk cells

In this experiment part of the culture was used to rebatch the mother fermenter and some daughter fermenters and part of the culture was used to freeze cells in bulk. Frozen bulk cells (total 14.4X10⁸ cells) were inoculated in a start culture in a 3 litre mother fermenter containing 5 g Cytodex per litre and EpiSerf medium, and thereafter incubated at 37 °C. Residual cryo-preservatives were removed by a medium change on day 1.
At day 2 trypsinisation was carried out, 50% of the cells were bulk frozen and the remaining cells were inoculated to micro-carriers in a subsequent fermenter.
From Table 1 it can be deduced that the cells do continue to grow at a normal rate between day 2 and 3
On day 4 the content of the mother fermenter was trypsin-detached and rebatched onto new micro-carriers (10 g/l) in two other fermenters next to the mother fermenter.
At day 5 the plating efficiency turned out to be about 85%.

**Table 1**

| **day** | **3 litre mother fermenter** | **3 litre fermenter** | **3 litre fermenter** |
|---|---|---|---|
| | **cells x 100.000/ml** | **cells x 100.000/ml** | **cells x 100.000/ml** |
| 0 | NOD | | |
| 1 | 6.6 | | |
| 2 | 14 | | |
| 3 | 15.5 | | |
| 4 | 30 | | |
| 5 | 5.5 | 10 | 10 |
| plating efficiency | 85% | 85% | 85% |

### EXAMPLE 5

### Transfer from small scale mother fermenter to large scale production fermenter

Cells were scaled up to a large scale in 65 litre and 550 litre fermenters (50 litre and 250 litre working volume, respectively) using a micro-carrier density of 5 g Cytodex per litre.
As can be seen from Table 2, 90% of the total of cells is transferred to the large scale fermenter from a 50 litre fermenter culture with 800.000 cells/ml of which 69% proved to be viable.
The same was found in the 50 litre mother fermenter, about 69% of the repropagating cells turned out to be viable.
The procedure was as follows:
On day 0, the carriers were allowed to settle in the 50 litre culture, where after the supernatant (culture medium) was removed and replaced by PBS. The content of the fermenter was agitated for 5-15 minutes. The supernatant was removed after resettling of the carriers. This step can be repeated if needed.
Next this step was repeated with PBS/EDTA (0.4 gram EDTA/litre PBS). Again the culture was agitated during 5-15 minutes, carriers were allowed to settle, the supernatant was removed, and the PBS/EDTA step was repeated until cells had become rounded and were ready to be trypsin-detached.
Then trypsin (0.025% final concentration) was added to the PBS/EDTA and incubated for 5-15 minutes. Next either the cell containing supernatant (after settling of now "nude" carriers) were transferred (as in example 9) or the mixture of cells plus carriers were transferred (total 80 % of total mix).
After transfer of the cells to the 550 litre fermenter the remainder of the cells (hence, 10% of the viable cells) were allowed to repopulate the carriers still present in the fermenter after refilling the 50 l fermenter with culture medium.
About 70% of the cells proved to be viable

**Table 2**

| **day** | **50 litre culture** | **250 litre culture** |
|---|---|---|
| | **cells x 100.000/ml** | **cells x 100.000/ml** |
| 0 | **8** (400 x 10⁸ total cells) | **1.1** (275 x 10⁸ viable cells) |
| 1 | | **0.8** |
| 2 | | **2.9** |
| 3 | | **3.4** |
| 4 | | **8.9** |
| 5 | | **18.0** |

### EXAMPLE 6

Analogous to Example 5, however, 80% of the culture of the carrier-bound cells were transferred from the mother bioreactor to the production bioreactor. Production was started after addition of virus.
The 20% of cells and carriers remaining in the mother bioreactor were trypsinized and detached and upon addition of new substrate into the mother bioreactor were allowed to repopulate the mother bioreactor while production is ongoing in the physically separated production bioreactor.

### EXAMPLE 7

### Large scale culture started from bulk frozen cells.

Bulk frozen cells were thawed and inoculated in a 10 litre (working volume) fermenter (Cytodex carrier density 5 g/l; culture medium EpiSerf) at an inoculation density of 1x10⁸ cells/ml. After attachment, the culture medium was replaced in order to remove residual cryo-protectants.
After day 1 the amount of viable cells attached to the carriers was 0.45x10⁶ cells/ml which from then on started growth. At a density of 2.8x10⁶ cells/ml the cells were detached from their carriers by trypsinisation and 80 % was transferred to a 50 litre working volume fermenter (carriers 5 g/l).
As can be deduced from Table 3, at day 1 the amount of viable cells after bulk freezing of cells was about 45 %.
Of the total amount of transferred cells, the viability after trypsin detachment was 71.4%.

**Table 3**

| **day** | **cell density (x 10⁶/l) in:** | |
|---|---|---|
| | **10 litre fermenter** | **50 litre fermenter** |
| 0 | **1.0** | |
| 1 /2 | **0.45** | |
| 3/4 | **1.3** | |
| 5 | **2.6** | |
| 6 | **2.8** (280 x 10⁸ total) | |
| 6 | **0.6** (60 x 10⁸ total) | **0.28** (140 x 10⁸ total) |
| 7 | | **0.4** (200 x 10⁸ total) |

## Claims

1. Method for the preparation of cells for use in the production of biologicals, by culturing cells up till a desired cell volume of a preproduction batch, where after in a repeated discontinuous process:
a) part of the cells of the preproduction batch is used for the preparation of at least one production batch, and
b) the remaining part of the cells of the preproduction batch is used as a seed for the preparation of at least one subsequent preproduction batch,
wherein the cells are anchorage-dependent cells which for their growing and/or propagation need to be attached to a substrate.

2. Method according to claim 1 wherein in the repeated discontinuous process:
a) part of the cells of the preproduction batch is transferred to be used for the preparation of at least one production batch, and
b) the remaining part of the cells of the preproduction batch is transferred to be used as a seed for the preparation of at least one subsequent preproduction batch.

3. Method according to claim 1 or 2, **characterised in that** a first preproduction batch is prepared from a working seed stock by at least one passage step.

4. Method according to claim 2, **characterised in that** the cells are grown on said substrate, and prior to each transfer step the cells are released from said substrate.

5. Method according to claim 1-4, **characterised in that** the biological of interest is a virus.

## Patentansprüche

1. Verfahren zur Herstellung von Zellen zur Verwendung bei der Produktion von biologischen Produkten durch Züchten von Zellen bis zu einem gewünschten Zellvolumen einer Präproduktions-Charge, wonach in einem wiederholten diskontinuierlichen Prozess:
a) ein Teil der Zellen der Präproduktions-Charge für die Herstellung von mindestens einer Produktionscharge verwendet wird, und
b) der übrige Teil der Zellen der Präproduktions-Charge als Keim für die Herstellung mindestens einer nachfolgenden Präproduktions-Charge verwendet wird,
wobei die Zellen verankerungsabhängige Zellen sind, die für ihr Wachstum und/oder ihre Vermehrung an einem Substrat befestigt sein müssen.

2. Verfahren nach Anspruch 1, wobei im wiederholten, diskontinuierlichen Prozess:
a) ein Teil der Zellen aus der Präproduktions-Charge transferiert wird, um für die Herstellung von mindestens einer Produktions-Charge verwendet zu werden, und
b) der übrige Teil der Zellen der Präproduktions-Charge transferiert wird, um als Keim für die Herstellung mindestens einer nachfolgenden Präproduktions-Charge verwendet zu werden.

3. Verfahren nach Anspruch 1 oder 2, **dadurch gekennzeichnet, dass** eine erste Präproduktions-Charge mittels mindestens eines Passagierungsschritts aus einer Arbeits-Stammlösung hergestellt wird.

4. Verfahren nach Anspruch 2, **dadurch gekennzeichnet, dass** die Zellen auf dem Substrat gezüchtet werden und vor jedem Transferschritt die Zellen vom Substrat freigegeben werden.

5. Verfahren nach Anspruch 1-4, **dadurch gekennzeichnet, dass** das biologische Produkt, an dem ein Interesse besteht, ein Virus ist.

## Revendications

1. Procédé de préparation de cellules à utiliser dans la production de produits biologiques, par culture des cellules jusqu'à un volume cellulaire désiré d'un lot de présérie, après quoi, dans un procédé discontinu répété :
a) une partie des cellules du lot de présérie est utilisée pour la préparation d'au moins un lot de production, et
b) la partie restante des cellules du lot de présérie est utilisée comme semence pour la préparation d'au moins un lot de présérie ultérieur,
dans lequel les cellules sont des cellules dépendantes d'un support qui, pour leur croissance et/ou leur propagation, doivent être fixées à un substrat.

2. Procédé selon la revendication 1, dans lequel, dans le procédé discontinu répété :
a) une partie des cellules du lot de présérie est transférée pour être utilisée dans la préparation d'au moins un lot de production, et
b) la partie restante des cellules du lot de présérie est transférée pour être utilisée comme semence pour la préparation d'au moins un lot de présérie ultérieur.

3. Procédé selon la revendication 1 ou.2, **caractérisé en ce qu'**un premier lot de présérie est préparé à partir d'un stock de semence de travail par au moins une étape de passage.

4. Procédé selon la revendication 2, **caractérisé en ce que** les cellules sont cultivées sur ledit substrat, et avant chaque étape de transfert, les cellules sont retirées dudit substrat.

5. Procédé selon les revendications 1-4, **caractérisé en ce que** le produit biologique en question est un virus.
